(19) Europäisches Patentamt
European Patent Office
Office européen des brevets

(11) EP 2 687 514 A1

(12) **EUROPEAN PATENT APPLICATION**

(43) Date of publication:
**22.01.2014 Bulletin 2014/04**

(51) Int Cl.:
***C07D 231/14*** *(2006.01)*

(21) Application number: **12176707.3**

(22) Date of filing: **17.07.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SOLVAY SA**
**1120 Bruxelles (BE)**

(72) Inventor: **Jaunzems, Janis**
**30559 Hannover (DE)**

(74) Representative: **Mross, Stefan P.M. et al**
**SOLVAY S.A.**
**Intellectual Assets Management**
**Rue de Ransbeek, 310**
**1120 Brussels (BE)**

(54) **Process for the preparation of derivatives of 1-substituted-3-fluoroalkyl-pyrazole-4-carboxylic acid in the presence of ammonium salt**

(57) A process for the manufacture of a compound of the general formula (I)

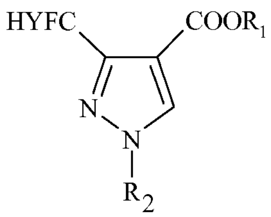

(I)

wherein
- Y is H, F, an alkyl group, a cycloalkyl group, an aralkyl group or an aryl group,
- $R^1$ is H or an organic residue,
- $R^2$ is H or an organic residue ;

which comprises submitting a compound of formula (II)

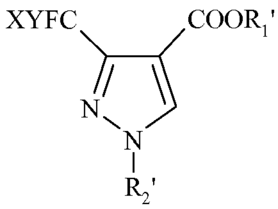

(II)

wherein Y is as defined above,
$R^{1,}$ is H or an organic residue,
$R^{2,}$ is H or an organic residue,
X is Cl, Br, I, CN, sulphate or sulphonate ;
to a reduction reaction in the presence of an ammonium salt.

EP 2 687 514 A1

## Description

The invention concerns a reduction process in the presence of ammonium salts for the manufacture of derivatives of 1-substituted-3-fluoroalkyl-pyrazole-4-carboxylic acid, in particular esters of 3-difluoromethyl-1-methyl-1H-pyrazole-4-carboxylic acid, which are useful e.g. as intermediates for pharmaceuticals and agrochemicals.

WO 2012/010692 describes the preparation of derivatives of 1-substituted-3-fluoroalkyl-pyrazole-4-carboxylic acid, which are intermediates for the manufacture of pyrazole carboxanilide fungicides, by a reduction process.

A problem faced when using the processes of the prior art is the over-reduction of the reaction product leading to a decrease in yield and to an increased effort for purification. Thus, there is a need for an improved process for the synthesis of derivatives of 1-substituted-3-fluoroalkyl-pyrazole-4-carboxylic acid which allows for high efficiency, high selectivity and for an environmental beneficial process.

The process according to this invention allows for a high selectivity with low degrees of over-reduction. It also allows for high efficiency, e.g. in terms of yield of the desired products. Furthermore, it is an environmentally or economically beneficial process. Additionally, it allows for an easier work-up.

The invention relates to a process for the manufacture of a compound of the general formula (I)

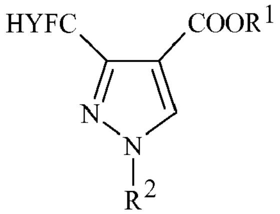

(I)

wherein

- Y is H, F, an alkyl group, a cycloalkyl group, an aralkyl group or an aryl group,
- $R_1$ is H or an organic residue,
- $R_2$ is H or an organic residue ;

which comprises submitting a compound of formula (II)

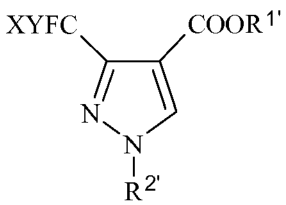

(II)

wherein

- Y is as defined above,
- X is Cl, Br, I, CN, sulphate or sulphonate,
- $R^{1'}$ is H or an organic residue,
- $R^{2'}$ is H or an organic residue ;

  to a reduction reaction in the presence of an ammonium salt of the general formula (III)

$$Z-N^+R^3R^4R^5R^6 \qquad (III)$$

  wherein
- $Z^-$ is an anion,
- $R^3$, $R^4$, $R^5$ and $R^6$ are independently selected from H, alkyl, cycloalkyl, aralkyl or aryl with the proviso that $R^3$, $R^4$, $R^5$ and $R^6$ are not all H.

In a preferred embodiment of the invention $R^1$ is methyl, ethyl or propyl, more preferably ethyl. In another preferred embodiment of the invention $R^2$ is methyl. In yet another preferred embodiment X is Cl and Y is F.

It should be noted that $R^{1'}$ can be identical to or different from $R^1$. Also $R^{2'}$ can be identical to or different from $R^2$. $R^{1'}$ and/or $R^{2'}$ are different from $R^1$ and $R^2$, if $R^{1'}$ and/or $R^{2'}$ undergo a reduction reaction in the process according to the invention. If reduction of the $R^{1'}$ and/or $R^{2'}$ occurs, the resulting $R^1$ and $R^2$ can be defined as the reduced groups of $R_1'$ and/or $R_2'$respectively.

The term "organic residue" is intended to denote in particular linear or branched alkyl or alkylene groups which may contain hetero atoms, such as in particular boron, silicon, nitrogen, oxygen or sulphur atoms and halogen atoms, cycloalkyl groups or cycloalkylene groups, heterocycles and aromatic systems. The organic residue may contain double or triple bonds and functional groups.

The organic residue comprises at least 1 carbon atom. It often comprises at least 2 carbon atoms. It preferably comprises at least 3 carbon atoms. More particularly preferably, it comprises at least 5 carbon atoms.

The organic residue generally comprises at most 100 carbon atoms. It often comprises at most 50 carbon atoms. It preferably comprises at most 40 carbon atoms. More particularly preferably, it comprises at most 30 carbon atoms.

The term "alkyl group" is intended to denote in particular a linear or branched alkyl substituent comprising from 1 to 20 carbon atoms, preferably 1, 2, 3, 4, 5, 6, 7, 8, 9 or 10 carbon atoms. Specific examples of such substituents are methyl, ethyl, n-propyl, isopropyl, n-butyl, isobutyl, tert-butyl, n-pentyl, isopentyl, n-hexyl, 2-hexyl, n-heptyl, n-octyl and benzyl. The alkyl group can optionally be substituted, e.g. by halogen.

The term "cycloalkyl group" is intended to denote in particular a substituent comprising at least one saturated carbocycle containing 3 to 10 carbon atoms, preferably 5, 6 or 7 carbon atoms. Specific examples of such substituents are cyclopropyl, cyclobutyl, cyclopentyl, cyclohexyl and cycloheptyl. The cycloalkyl group can optionally be substituted, e.g. by halogen.

The term "aryl group" is intended to denote an optionally substituted group which derives from an aromatic nucleus such as, in particular, a C6-C10 aromatic nucleus, in particular phenyl or naphthyl. The aryl group can optionally be substituted, e.g. by halogen, alkyl, or cycloalkyl.

The term "aralkyl group" is intended to denote a group which derives from an alkylene group bound to an aryl group as defined above. The aryl group can optionally be substituted, e.g. by halogen, alkyl, or cycloalkyl.

When the organic residue contains one or optionally more double bonds, it is often chosen from an alkenyl group comprising from 2 to 20 carbon atoms, preferably 2, 3, 4, 5, 6, 7, 8, 9 or 10 carbon atoms or a cycloalkenyl group comprising from 3 to 20 carbon atoms, preferably 3, 4, 5, 6, 7, 8, 9 or 10 carbon atoms. Specific examples of such groups are vinyl, 1-allyl, 2-allyl, n-but-2-enyl, isobutenyl, 1,3-butadienyl, cyclopentenyl, cyclohexenyl and styryl.

When the organic residue contains one or optionally more triple bonds, it is often chosen from an alkinyl group comprising from 2 to 20 carbon atoms, preferably 2, 3, 4, 5, 6, 7, 8, 9 or 10 carbon atoms. Specific examples of such groups are ethinyl, 1-propinyl, 2-propinyl, n-but-2-inyl and 2-phenylethinyl.

When the organic residue contains one or optionally more aromatic systems, it is often an aryl group comprising from 6 to 24 carbon atoms, preferably from 6 to 12 carbon atoms. Specific examples of such groups are phenyl, 1-tolyl, 2-tolyl, 3-tolyl, xylyl, 1-naphthyl and 2-naphthyl.

The term "heterocycle" is intended to denote in particular a cyclic system comprising at least one saturated or unsaturated ring made up of 3, 4, 5, 6, 7 or 8 atoms, at least one of which is a hetero atom. The hetero atom is often chosen from B, N, O, Si, P and S. It is more often chosen from N, O and S.

Specific examples of such heterocycles are aziridine, azetidine, pyrrolidine, piperidine, morpholine, 1,2,3,4-tetrahydroquinoline, 1,2,3,4-tetrahydroisoquinoline, perhydroquino line, perhydroisoquino line, isoxazolidine, pyrazoline, imidazoline, thiazoline, tetrahydrofuran, tetrahydrothiophene, pyran, tetrahydropyran and dioxane.

The organic residues as defined above may be unsubstituted or substituted with functional groups. The term "functional group" is intended to denote in particular a substituent comprising or consisting of a hetero atom. The hetero atom is often chosen from B, N, O, Al, Si, P, S, Sn, As and Se and the halogens. It is more often chosen from N, O, S and P, in particular N, O and S.

The functional group generally comprises 1, 2, 3, 4, 5 or 6 atoms.

By way of functional groups, mention may, for example, be made of halogens, a hydroxyl group, an alkoxy group, a mercapto group, an amino group, a nitro group, a carbonyl group, an acyl group, an optionally esterified carboxyl group, a carboxamide group, a urea group, a urethane group and the thiol derivatives of the abovementioned groups containing a carbonyl group, phosphine, phosphonate or phosphate groups, a sulphoxide group, a sulphone group and a sulphonate group.

In a preferred embodiment of the process according to the invention, $R^1$ is H, $C_1$-$C_8$-alkyl, $C_3$-$C_8$-cycloalkyl, $C_1$-$C_4$-alkoxy-$C_1$-$C_4$-alkyl, $C_3$-$C_8$-cycloalkoxy-$C_1$-$C_4$-alkyl, $C_2$-$C_8$-alkenyl or is benzyl which is optionally substituted by 1,2 or 3 substituents $R^{Y1}$ independently of one another selected from the group consisting of $C_1$-$C_4$-alkyl, $C_1$-$C_4$-alkoxy and nitro ; and

$R^2$ is hydrogen, $C_1$-$C_4$-alkyl, benzyl or phenyl, where the two last-mentioned substituents may be unsubstituted or optionally substituted by 1,2 or 3 substituents $R^{Y2}$ independently of one another selected from the group consisting

of halogen, nitrile, nitro, $C_1$-$C_4$-alkyl, $C_1$-$C_4$-haloalkyl, $C_1$-$C_4$-alkoxy and $C_1$-$C_4$-haloalkoxy ; and X is Cl.

The terms, used in the definition of the variables, for organic groups, such as, for example, the term "halogen", are collective terms representing the individual members of these groups of organic moieties.

The prefix $C_x$-$C_y$ denotes the number of possible carbon atoms in the case in question. $C_1$-$C_4$-Alkyl includes, for example, methyl, ethyl, propyl, 1-methylethyl, butyl, 1-methylpropyl, 2-methylpropyl or 1,1-dimethylethyl.

The term "halogen" denotes in each case fluorine, bromine, chlorine or iodine, especially fluorine, chlorine or bromine.

The term "$C_1$-$C_4$-alkoxy-$C_1$-$C_4$-alkyl", as used herein, describes $C_1$-$C_4$-alkyl radicals where one carbon atom is attached to a $C_1$-$C_4$-alkoxy radical. Examples of these are $CH_2$-$OCH_3$, $CH_2$-$OC_2H_5$, n-propoxymethyl, $CH_2$-$OCH(CH_3)_2$, n-butoxymethyl, (1-methylpropoxy)methyl, (2-methylpropoxy)methyl, $CH_2$-$OC(CH_3)_3$, 2-(methoxy)ethyl, 2-(ethoxy)ethyl, 2-(n-propoxy)ethyl, 2-(1-methylethoxy)ethyl, 2-(n-butoxy)ethyl, 2-(1-methylpropoxy)ethyl, 2-(2-methylpropoxy)ethyl, 2-(1,1-dimethylethoxy)ethyl, 2-(methoxy)propyl, 2-(ethoxy)propyl, 2-(n-propoxy)propyl, 2-(1-methylethoxy)propyl, 2-(n-butoxy)propyl, 2-(1-methylpropoxy)propyl, 2-(2-methylpropoxy)propyl, 2-(1,1-dimethylethoxy)propyl, 3-(methoxy)propyl, 3-(ethoxy)propyl, 3-(n-propoxy)propyl, 3-(1-methylethoxy)propyl, 3-(n-butoxy)propyl, 3-(1-methylpropoxy)propyl, 3-(2-methylpropoxy)propyl, 3-(1,1-dimethylethoxy)propyl, 2-(methoxy) butyl, 2-(ethoxy)butyl, 2-(n-propoxy)butyl, 2-(1-methylethoxy)butyl, 2-(n-butoxy)butyl, 2-(1-methylpropoxy)butyl, 2-(2-methylpropoxy)butyl, 2-(1,1-dimethylethoxy)butyl, 3-(methoxy)butyl, 3-(ethoxy)butyl, 3-(n-propoxy)butyl, 3-(1-methylethoxy)butyl, 3-(n-butoxy)butyl, 3-(1-methylpropoxy)butyl, 3-(2-methylpropoxy)butyl, 3-(1,1-dimethylethoxy)butyl, 4-(methoxy)butyl, 4-(ethoxy)butyl, 4-(n-propoxy)butyl, 4-(1-methylethoxy)butyl, 4-(n-butoxy)butyl, 4-(1-methylpropoxy)butyl, 4-(2-methylpropoxy) butyl, 4-(1,1-dimethylethoxy)butyl, etc.

The term "$C_2$-$C_8$-alkenyl", as used herein, describes straight-chain and branched unsaturated hydrocarbon radicals having 2 to 8 carbon atoms and at least one carbon-carbon double bond, such as, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl, 1-heptenyl, 2-heptenyl, 1-octenyl or 2-octenyl.

In the process according to the invention, the reduction reaction of the compound of formula (II) can be carried out according to different reduction reactions.

In a preferred embodiment, the reduction process according to the invention is a hydrogenation reaction comprising reacting the compound of formula (II) with hydrogen, in particular hydrogen gas in the presence of a hydrogenation catalyst. The use of hydrogen for said reduction reaction advantageously avoids the formation of waste.

In this embodiment of the process according to the invention, the hydrogenation reaction is preferably carried out in the liquid phase. In this case, the compound of formula (II) is advantageously dissolved in a solvent. Solvents which can be used in the hydrogenation reaction are chosen, for example, from polar solvents. In general, polar solvents comprising at least one OH group are highly suitable. Examples of polar solvents comprising at least one OH group may be selected from a group consisting of aliphatic alcohols preferably comprising from 1 to 3 carbon atoms, water, organic acids such as acetic acid, aqueous solutions of acids, preferably inorganic acids. Aliphatic alcohols preferably comprising from 1 to 3 carbon atoms such as methanol, ethanol, isopropanol (IPA) and the like are preferred. A single polar solvent can be used or a mixture of several polar solvents.

The hydrogenation reaction is generally carried out in the presence of a hydrogenation catalyst. The hydrogenation catalyst is advantageously chosen from the metals from Group VIII of the Periodic Table of the Elements (IUPAC 1970). Mention will be made in particular of a catalyst comprising at least one metal chosen from palladium, platinum and rhodium. A catalyst comprising palladium is preferred. Optionally, the reduction process comprises substituting halogen, in particular chlorine, by hydrogen and simultaneously hydrogenating of at least one of the unsaturated bonds in the substituents $R_1$ and/or $R_2$.

The hydrogenation catalyst is often a supported catalyst. Supports which can be used are chosen, for example, from alumina, silica and carbon in particular active carbon or charcoal. A catalyst supported on active carbon gives good results. An example of a suitable catalyst comprises palladium on carbon support, often referred to as Pd/C.

When the hydrogenation catalyst is a supported catalyst comprising a metal from Group VIII, the metal content

is generally at least 0.1 % by weight with respect to the total weight of the catalyst. The metal content is often greater than or equal to 1 % by weight. Preferably, the metal content is greater than or equal to 5 % by weight. The metal content is generally at most 50 % by weight with respect to the total weight of the catalyst.

In a very particularly preferred way, the catalyst is supported palladium, preferably supported on a support as described above, preferably exhibiting a metal content as described above.

In the hydrogenation reaction, the temperature of the reaction is generally at least -10°C. The temperature of the reaction is often at least 0°C. Preferably, this temperature is at least 20°C. The temperature of the reaction is generally at most 160°C. The temperature of the reaction is often at most 150°C. Preferably, this temperature is at most 140°C. A temperature of at most 130°C is very particularly preferred.

In the hydrogenation reaction, the pressure of the reaction is generally at least 1 bar absolute. Preferably, the pressure is at least 1.5 bar. The pressure of the hydrogenation reaction is generally at most 30 bar absolute. Preferably, the pressure is at most 20 bar. In a particularly preferred way, the pressure is from 1 to 15 bar.

In a particular embodiment, the hydrogenation reaction is carried out at a temperature from 0°C to 150°C and a pressure from 1 bar to 15 bar. Preferably, the hydrogenation reaction is carried out at a temperature from 40°C to 80°C and a pressure from 1 bar to 15 bar.

When use is made of hydrogen as hydrogenation reactant, the molar ratio of hydrogen to the compound of formula (II) is generally greater than or equal to 1. This ratio is generally at most 1000. Preferably, this ratio is at most 100. More preferably, this ratio is at most 10.

In the hydrogenation reaction, the concentration of the compound of formula II in the reaction medium is generally at least 5 % by weight with respect to the total weight of the reaction medium. This concentration is often at least 10 % by weight. Preferably, the concentration is at least 20 % by weight. The concentration of the compound of formula II in the reaction medium is generally at most 50 % by weight with respect to the total weight of the reaction medium.

Optionally, the reduction reaction can be performed in the presence of additional additives. An example for an additional additive is CsF.

Surprisingly, it has been found that the reduction reaction of the present invention in the presence of an ammonium salt of the general formula (III) $Z^-N^+R^3R^4R^5R^6$ (III) is particularly suitable for selectively substituting a halogen atom, in particular a chlorine atom, by a hydrogen atom while the pyrazole ring as well as the fluoro substituent in the 3-substituent remain substantially unaffected. In a preferred embodiment of the invention, Z is F, Cl, Br, OH or $BF_4$, more preferably Z is F or Cl. In another preferred embodiment of the invention, $R^3$, $R^4$ $R^5$ and $R^6$ are independently selected from C1-C8 alkyl, more preferably $R^3$, $R^4$, $R^5$ and $R^6$ are methyl, ethyl, butyl or octyl. Most preferably, the invention is carried out in presence of tetrabutylammonium fluoride, tetrabutylammonium chloride, tetramethylammonium fluoride, tetramethylammonium chloride, tetraoctylammonium chloride, or tetrabutylammonium tetrafluoroborate.

Another aspect of the invention is a process for the manufacture of an agrochemically or pharmaceutically active compound comprising

a) the manufacture of a compound of the general formula (I)

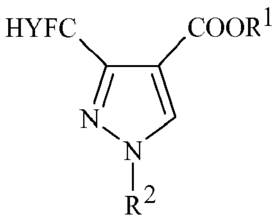

(I)

wherein

- Y is H, F, an alkyl group, a cycloalkyl group, an aralkyl group or an aryl group,
- $R^1$ is H or an organic residue,
- $R^2$ is H or an organic residue ;
  which comprises submitting a compound of formula (II)

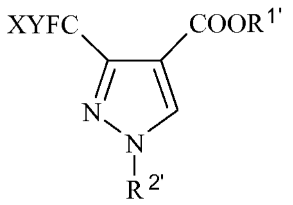

(II)

wherein Y is as defined above,
$R^{1'}$ is H or an organic residue,
$R^{2'}$ is H or an organic residue,
X is Cl, Br, I, CN, sulphate or sulphonate,
to a reduction reaction ; and

b) submitting the crude product mixture obtained in step a) to a subsequent synthetic step ;

wherein no purification step of the crude product mixture is performed between step a) and step b).

In a preferred embodiment of this aspect of the invention the crude product mixture comprises a compound of general formula (IV) :

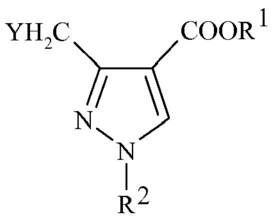

(IV)

wherein $R^1$ and $R^2$ are independently H or an organic residue, in a concentration of equal to or below 2 wt %, preferably equal to or below 1 wt %, more preferably equal to or below 0.5 wt %.
"Crude product mixture" is intended to denote the residue after completion of the reduction reaction of the invention including subsequent filtration and evaporation of the solvent(s) as well as other volatiles without a subsequent purification step.

"Further purification step" is intended to denote e.g. distillation, sublimation, crystalisation, trituration, or re-dissolving/precipitation.

Should the disclosure of any patents, patent applications, and publications which are incorporated herein by reference conflict with the description of the present application to the extent that it may render a term unclear, the present description shall take precedence.

The following examples are intended to further explain the invention without limiting it.

**Examples**

General procedure for the reduction reaction yielding ethyl 1-methyl-3-difluoromethyl-pyrazole-4-carboxylate :

To a solution of 100 mg ethyl 1-methyl-3-chlorodifluoromethyl-pyrazole-4-carboxylate (0.4 mmol) in 3 ml THF was added the ammonium salt, 35 mg $K_2CO_3$ (0.25 mol), optionally the further additive CsF, and 20 mg 5 % palladium on charcoal. The resulting mixture was heated under vigorous stirring under hydrogen atmosphere in a steel reactor. The mixture was cooled down. The solids were filtered off and washed with a small amount of THF. The solvent was evaporated to yield a crude product mixture.

Table 1 :

| # | Ammonium salt, amount | Amount (CsF) | T (°C) / t (h) / $p_{H2}$ (bar) | Yield (GC %) | Selectivity |
|---|---|---|---|---|---|
| 1 | $N^+Bu_4\ (BF_4)^-$, 10 mg | 12 mg | 110/2/11 | 58 | 16 |
| 2 | $N^+(n\text{-octyl})_4\ Cl^-$, 10mg | 12 mg | 110/20/11 | 72 | 24.6 |

(continued)

| # | Ammonium salt, amount | Amount (CsF) | T (°C) / t (h) / $p_{H2}$ (bar) | Yield (GC %) | Selectivity |
|---|---|---|---|---|---|
| 3 | $N^+Bu_4\ Cl^-$, 10 mg | 12 mg | 110/2/11 | 37 | 17 |
| 4 | $N^+Et_4\ Br^-$, 10 mg | 12 mg | 110/2/11 | 38 | 20.5 |
| 5 | $N^+Bu_4F^-$, 10mg | 12 mg | 110/15/11 | 91 | 14 |
| 6 | $N^+Me_4F^- \cdot 4\ H_2O$, 5 mg | 12 mg | 110/1/11 | 94 | 16.5 |
| 7 | $N^+Me_4\ Cl^-$, 5 mg | - | 110/1/11 | 63 | 12 |
| 8 | TMAF $\cdot$ $4H_2O$ 5 mg | - | 110/1/11 | 97 | 32 |
| A | none | 12 mg | 110/1/11 | 88 | 8.2 |

The reduction reaction of ethyl 1-methyl-3-chlorodifluoromethyl-pyrazole-4-carboxylate was carried out in an autoclave at different temperatures, different pressures and different reaction times in the presence of different ammonium salts and optionally the further additive CsF as summarized in table 1. Yield were determined by GC (peak %). The selectivity is given by the following formula :

Selectivity (%) = yield % (ethyl 1-methyl-3-difluoromethyl-pyrazole-4-carboxylate) / [yield % (ethyl 1-methyl-3-monofluoromethyl-pyrazole-4-carboxylate) + yield % (ethyl 1-methyl-3-methyl-pyrazole-4-carboxylate)

Row A is a comparative example with the no addition of an ammonium salt.

The results in table 1 show that the process of the invention in the presence of ammonium salts allows for a surprising improvement of selectivity as compared to the comparative example A. Surprisingly, it has been found that these results are maintained even in the absence of an additional additive like CsF allowing for a great cost reduction of the process. Also surprisingly, it has been found that the ammonium salt used in the process of the invention remains bound to the Palladium catalyst during work-up and is almost not detectable in the product-containing filtrate allowing for a great improvement in the ease of work-up of the process mixture.

## Claims

1. A process for the manufacture of a compound of the general formula (I)

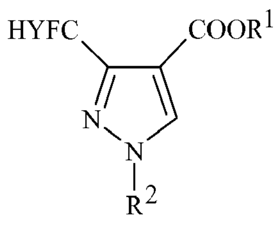

(I)

wherein

- Y is H, F, an alkyl group, a cycloalkyl group, an aralkyl group or an aryl group,
- $R^1$ is H or an organic residue,
- $R^2$ is H or an organic residue ;

which comprises submitting a compound of formula (II)

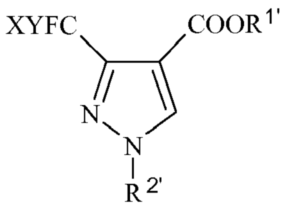

(II)

wherein Y is as defined above,
$R^{1'}$ is H or an organic residue,
$R^{2'}$ is H or an organic residue,
X is Cl, Br, I, CN, sulphate or sulphonate,
to a reduction reaction in the presence of an ammonium salt of the general formula (III)

$Z^-N^+R^3R^4R^5R^6$ (III)

wherein

- $Z^-$ is an anion,
- $R^3$, $R^4$, $R^5$ and $R^6$ are independently selected from H, alkyl, aralkyl or aryl with the proviso that $R^3$, $R^4$, $R^5$ and $R^6$ are not all H.

**2.** The process according to claim 1, wherein $R^1$ is methyl, ethyl or propyl, preferably $R^1$ is ethyl.

**3.** The process according to claim 1 or 2, wherein $R^2$ is methyl.

**4.** The process according to any one of claims 1 to 3, wherein X is Cl and YisF.

**5.** The process according to any one of the claims 1 to 4 wherein $R^1$ is ethyl and $R^2$ is methyl.

**6.** The process according to anyone of claims 1 to 5, wherein the reduction is carried out with hydrogen in the presence of a hydrogenation catalyst, preferably in the presence of palladium, more preferably in the presence of palladium on a support.

**7.** The process according to any one of claims 1 to 6, wherein Z is F, Cl, Br, OH or $BF_4$, preferably Z is F or Cl.

**8.** The process according to any one of claims 1 to 7, wherein $R^3$, $R^4$ $R^5$ and $R^6$ are independently selected from C1-C8 alkyl.

**9.** The process according to any one of claims 1 to 8, wherein $R^3$, $R^4$ $R^5$ and $R^6$ are methyl, ethyl, butyl, or octyl.

**10.** The process according to any one of claims 1 to 9, wherein the ammonium salt of the general formula (III) is tetrabutylammonium fluoride, tetrabutylammonium chloride, tetramethylammonium fluoride, tetramethylammonium chloride, tetraoctylammonium chloride, or tetrabutylammonium tetrafluoroborate.

**11.** The process according to any one of claims 1 to 10, wherein the temperature of the reaction is from 40 to 80°C.

**12.** The process according to any one of claims 1 to 11, wherein the pressure of the reaction is from 1 to 15 bar.

**13.** A process for the manufacture of an agrochemically or pharmaceutically active compound comprising

a) the manufacture of a compound of the general formula (I)

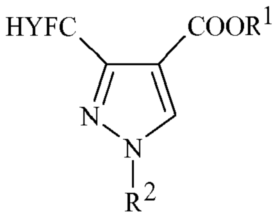

(I)

wherein

- Y is H, F, an alkyl group, a cycloalkyl group, an aralkyl group or an aryl group,
- $R^1$ is H or an organic residue,
- $R^2$ is H or an organic residue ;

which comprises submitting a compound of formula (II)

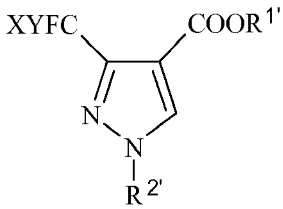

(II)

wherein Y is as defined above,
$R^{1'}$ is H or an organic residue,
$R^{2'}$ is H or an organic residue,
X is Cl, Br, I, CN, sulphate or sulphonate,
to a reduction reaction ; and
b) submitting the crude product mixture obtained in step a) to a subsequent synthetic step ;
wherein no purification step of the crude product mixture is performed between step a) and step b).

**14.** The process according to claim 13 wherein the crude product mixture comprises a compound of general formula (IV) :

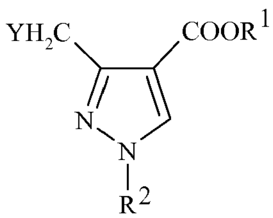

(IV)

wherein $R^1$ and $R^2$ are independently H or an organic residue,
in a concentration of equal to or below 2 wt %, preferably equal to or below 1 wt %, more preferably equal to or below 0.5 wt %.

Europäisches Patentamt
European Patent Office
Office européen des brevets

# EUROPEAN SEARCH REPORT

Application Number
EP 12 17 6707

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A,D | WO 2012/010692 A1 (SOLVAY [BE]; BRAUN MAX JOSEF [DE]; JAUNZEMS JANIS [DE]) 26 January 2012 (2012-01-26) * the whole document * ----- | 1-12 | INV. C07D231/14 |
| | | | TECHNICAL FIELDS SEARCHED (IPC) |
| | | | C07D |

~~The present search report has been drawn up for all claims~~

1

| Place of search | Date of completion of the search | Examiner |
|---|---|---|
| Munich | 17 October 2012 | Fink, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

Application Number
EP 12 17 6707

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

Application Number
EP 12 17 6707

The Search Division considers that the present European patent application does not comply with the requirements of unity of invention and relates to several inventions or groups of inventions, namely:

1. claims: 1-12

   the process for the preparation of a 1-substituted-3-fluoroalkyl-pyrazole-4-carboxylic acid derivative of the present formula (I);

   ---

2. claims: 13, 14

   the process for the preparation of an agrochemically or pharmaceutically active compound according to the present independent claim 13;

   ---

## ANNEX TO THE EUROPEAN SEARCH REPORT ON EUROPEAN PATENT APPLICATION NO. EP 12 17 6707

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2012

| Patent document cited in search report | Publication date | Patent family member(s) | Publication date |
|---|---|---|---|
| WO 2012010692 A1 | 26-01-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012010692 A